# EUROPÄISCHE PATENTSCHRIFT

(11) **EP 2 179 708 B1**
(45) Veröffentlichungstag und Bekanntmachung des Hinweises auf die Patenterteilung: **26.01.2011**
(21) Anmeldenummer: 09010114.8
(22) Anmeldetag: 05.08.2009
(51) Int. Cl.: A61F 2/64, A61F 2/68, A61F 2/00, A61F 2/50

(54) **Störungsfreies Sicherheits-Prothesengelenk**
Error-free safety prosthesis joint
Articulation prothétique de sécurité sans déficience

(30) Priorität: 22.10.2008 TW 97218845 U
(43) Veröffentlichungstag der Anmeldung: 28.04.2010
(73) Patentinhaber: medi GmbH & Co. KG, 95448 Bayreuth (DE)
(72) Erfinder: Kremser, Dirk, 95444 Bayreuth (DE); Shen, Hsin Fa, Banqiao City Taipei County 220 (TW)
(74) Vertreter: Blaumeier, Jörg

(56) Entgegenhaltungen:
- EP-A- 1 166 726
- DE-U1- 20 119 049
- DE-U1- 29 521 138

## Beschreibung

Die Erfindung betrifft ein störungsfreies Sicherheits-Prothesengelenk, insbesondere ein Prothesengelenk für Körperbehinderte. Das Dokument DE 201 19 049 U stellt der nächste Stand der Technik dar.

Um das Gehproblem des Körperbehinderten zu verbessern, sind Prothesengelenke unterschiedlicher Bauarten entwickelt worden. Das Prothesengelenk wirkt wie ein Knie. Das heißt, dass das Prothesengelenk oben mit dem Oberschenkel und unten mit dem Unterschenkel verbunden ist, mit dem Ziel, dass das Unterschenkel-Prothesengelenk beim Gehen so gebeugt werden kann, dass ein möglichst natürliches Gehen erzielt wird. Hierdurch ergibt sich eine erhöhte Gehflexibilität.

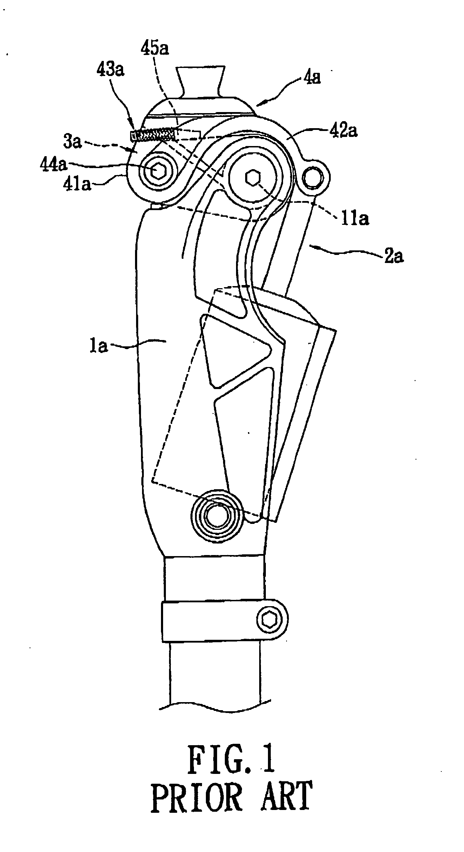

Wie in Fig. 1 gezeigt, weist ein herkömmliches Prothesengelenk eine Unterschenkel-Verbindungsvorrichtung 1a, eine Puffervorrichtung 2a, eine Bremsvorrichtung 3a und einen Oberdeckel 4a auf. Die Bremsvorrichtung 3a ist hinten auf einen am oberen Ende der Unterschenkel-Verbindungsvorrichtung 1a angeordneten Befestigungszapfens 11 a aufgesteckt. Der Oberdeckel 4a dient zur Verbindung des Prothesenschaftes. Der Oberdeckel 4a umfasst einen Vorderarm 41a, einen Hinterarm 42a und eine Einstellvorrichtung 43a. Der Vorderarm 41a ist mittig mit einem querverlaufenden Zapfen 44a versehen, der durch das vordere Ende der Bremsvorrichtung 3a hindurchgeht. Der Hinterarm 42a ist schwenkbar am oberen Ende der Puffervorrichtung 2a angeordnet. Zwischen dem oberen Ende der vorderen Seite der Bremsvorrichtung 3a und dem Oberdeckel 4a liegt noch ein Spalt vor.

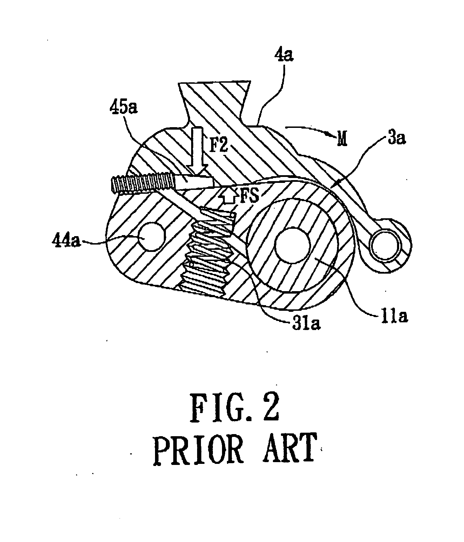

Beim Stehen des Körperbehinderten erzeugt der Oberschenkel eine nach unten gerichtete Kraft F2 [siehe Fig. 2], wobei der Oberdeckel 4a um den Zapfen 44a schwenkt und somit ein Betätigungsmoment M im Uhrzeigersinn erzeugt. Es wird ein Keilstück 45a der Einstellvorrichtung 43a nach unten gegen die Bremsvorrichtung 3a gedrückt und der Befestigungszapfen 11a eingeklemmt. Damit wird vermieden, dass das Prothesengelenk einbeugt. So kann der Körperbehinderte problemlos stehen.

Es wird dann auf Fig. 2 Bezug genommen. Beim Belasten des Prothesengelenks dient der Zapfen 44a als Drehachse. Auf diese Weise wird die Bremsvorrichtung 3a leicht nach unten verschoben, um eine Bremswirkung zu erzeugen. Es ist üblich, dass die Bremsvorrichtung 3a mit einer Feder 31 a zur Bereitstellung einer Aufspannkraft FS versehen ist, die als Gegenkraft gegen das Bremsverhalten einwirkt. Wird die Aufspannkraft der Feder 31a für das normale Gehen eingestellt, kommt es beim schnellen Gehen zu Schwierigkeiten. Das Schwenkmoment vergrößert sich beim schnellen Gehen, wodurch das Prothesengelenk nicht mehr einbeugt.

Beim oben erwähnten herkömmlichen Prothesengelenk tritt ein Kompromissproblem zwischen der Straffheit und der Flexibilität des Prothesengelenks auf. Insbesondere ist dieses für intensive und schnelle Bewegung ungeeignet. Wird das körperliche Gewicht vom Prothesenunterschenkel gestützt, wird der Oberdeckel 4a unter Einwirkung eines entgegenwirkenden Drehmoments geschwenkt. Unterschreitet das aus der Gewichtskraft resultierende Drehmoment das entgegenwirkende Drehmoment, kann es zu einer abnormalen Einbeugung des Prothesengelenks kommen und somit die Anwendungssicherheit beeinträchtigen.

Der Erfindung liegt die Aufgabe zugrunde, ein störungsfreies Sicherheits-Prothesengelenk zu schaffen, das dem Körperbehinderten eine erhöhte und sichere Gehflexibilität ermöglicht.

Diese Aufgabe wird erfindungsgemäß gelöst durch ein störungsfreies Sicherheits-Prothesengelenk, das die im Anspruch 1 angegebenen Merkmale aufweist. Weitere vorteilhafte Weiterbildungen der Erfindung gehen aus den Unteransprüchen hervor.

Gemäß der Erfindung wird ein störungsfreies Sicherheits-Prothesengelenk geschaffen, das aufweist:
eine Unterschenkel-Verbindungsvorrichtung, die einen Hauptkörper, einen Sockel, ein Antriebselement und eine Rockstelleinheit umfasst, wobei ein Aufnahmeraum von der Innenwand des Hauptkörpers begrenzt wird, und wobei sich der Sockel ausgehend von einem Ende des Hauptkörpers erstreckt, und wobei die Rückstelleinheit im Aufnahmeraum vorgesehen ist, und wobei ein Ende der Rückstelleinheit mit dem Antriebselement verbunden ist, während das andere Ende desselben schwenkbar mit dem Hauptkörper verbunden ist;
eine Oberschenkel-Verbindungsvorrichtung, die einen Verbindungsabschnitt und einen Sockelabschnitt besitzt, wobei der Verbindungsabschnitt an seinem einen Ende mit einem Oberschenkelarretierelement versehen ist, während der Sockelabschnitt an einem anderen Ende des Verbindungsabschnitts angeordnet ist, und wobei die Rückstelleinheit an einem Ende des Antriebselements befestigt ist, während der Sockelabschnitt schwenkbar an dem anderen Ende desselben angeordnet ist;
ein Bremselement, das im Aufnahmeraum vorgesehen und am Oberschenkel-Verbindungsvorrichtung befestigt ist;
ein Bremsbügel, der sich im Aufnahmeraum befindet, wobei der Bremsbügel ein erstes Zapfenloch und einen ersten Befestigungsabschnitt aufweist, und wobei der erste Befestigungsabschnitt mit einem dritten Arretierloch versehen ist, das mit dem Bremselement verbunden ist; und
einen Bremszapfen, der durch das Zapfenloch hindurch verläuft und an der Unterschenkel-Verbindungsvorrichtung befestigt ist.

Zusammengefasst lassen sich mit dem erfindungsgemäßen störungsfreien Sicherheits-Prothesengelenk beispielsweise folgende Vorteile realisieren:
1. Befindet sich der Massenschwerpunkt des Körperbehinderten hinter dem störungsfreien Sicherheits-Prothesengelenk, liegt das Bremselement am Bremsbügel so an, dass die Begrenzungsfläche des ersten Zapfenlochs gegen die Außenwand des Bremszapfens gedrückt wird. Auf diese Weise tritt keine abnormale Abbiegung des Prothesengelenks auf. Damit ist eine sichere Verwendung gewährleistet.
2. Befindet sich der Massenschwerpunkt des Körperbehinderten vor dem störungsfreien Sicherheits-Prothesengelenk, ist der Bremsbügel um den Bremszapfen schwenkbar. Damit ist eine störungsfreie Anwendung gewährleistet. Außerdem wird dem Körperbehinderten eine erhöhte Gehflexibilität ermöglicht.

Im Folgenden werden die Erfindung und deren Ausgestaltungen anhand der Zeichnung näher erläutert. In der Zeichnung zeigt:
- Fig. 1: eine Seitenansicht eines herkömmlichen Prothesengelenks;
- Fig. 2: eine schematische Darstellung der Einwirkung der Kraft einer Druck- vorrichtung des herkömmlichen Prothesengelenks;
- Fig. 3: eine perspektivische Explosionsdarstellung eines erfindungsgemä- ßen Prothesengelenks;
- Fig. 4: eine perspektivische Zusammenbaudarstellung eines erfindungsge- mäßen Prothesengelenks;
- Fig. 5: eine schematische Darstellung der Einwirkung der Kraft eines Bremselements und eines Bremsbügels des erfindungsgemäßen Prothesengelenks;
- Fig. 6: einen Schnitt durch das Bremselement und den Bremsbügel des er- findungsgemäßen Prothesengelenks in einem störungsfreien Zu- stand;
- Fig. 7: einen Schnitt durch das Bremselement und den Bremsbügel des er- findungsgemäßen Prothesengelenks in einem weiteren störungsfrei- en Zustand;
- Fig. 8: eine perspektivische Explosionsdarstellung eines weiteren Ausfüh- rungsbeispiels eines erfindungsgemäßen Prothesengelenks; und
- Fig. 9: eine schematische Darstellung der Einwirkung der Kraft eines Bremselements und eines Bremsbügels des weiteren Ausführungs- beispiels eines erfindungsgemäßen Prothesengelenks nach Fig. 8;

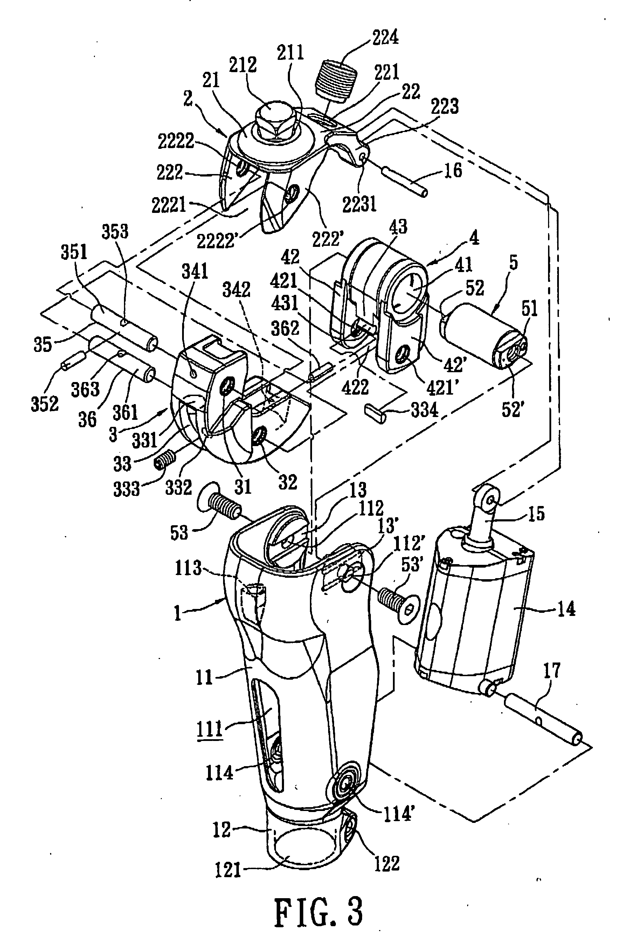

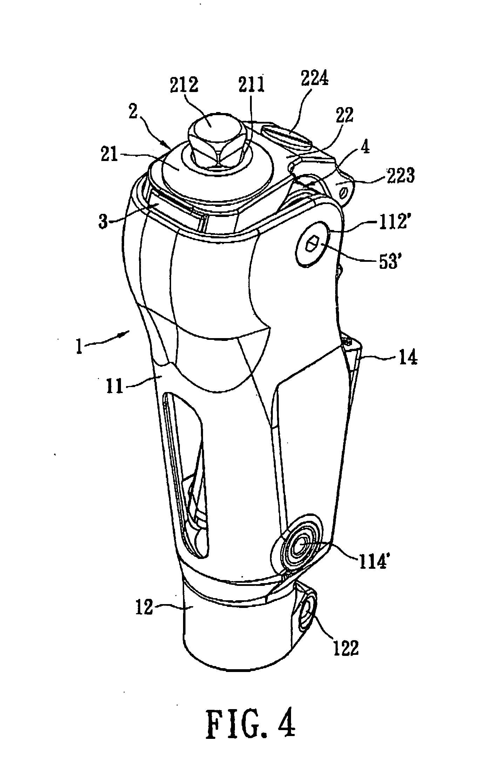

Bezug nehmend auf die Fig. 3 bis Fig. 4 weist ein erfindungsgemäßes störungsfreies Sicherheits-Prothesengelenk eine Unterschenkel-Verbindungsvorrichtung 1, eine Oberschenkel-Verbindungsvorrichtung 2, ein Bremselement 3, einen Bremsbügel 4 und einen Bremszapfen 5 auf.

Die Unterschenkel-Verbindungsvorrichtung 1 besitzt einen im Wesentlichen U-förmigen, dünnen Hauptkörper 11, wobei sich der Sockel 12 ausgehend von einem Ende des Hauptkörpers 11 erstreckt Der Sockel 12 weist eine Unterschenkel-Befestigungsöffnung 121 und eine Unterschenkel-Arretiernut 122 auf. Die Unterschenkel-Befestigungsöffnung 121 dient der Aufnahme eines Prothesenunterschenkels, der mit der Unterschenkel-Arretiernut 122 an der Unterschenkel-Verbindungsvorrichtung 1 arretierbar ist. Ein Aufnahmeraum 111 wird von der Innenwand des Hauptkörpers 11 begrenzt, wobei eine Rückstelleinheit 14 im Aufnahmeraum 111 aufnehmbar ist. Die Rückstelleinheit 14 kann als Rückstellfeder ausgeführt sein. Alternativ dazu kann diese als Kolbeneinheit ausgeführt sein, die aus einem Zylinder, einem Druckkolben und einer Einstellunterkappe besteht. Die Rückstelleinheit 14 ist oben mit einem Antriebselement 15 verbunden. Der Hauptkörper 11 ist oben an seinen beiden Seiten mit ein Paar von einander gegenüberliegenden zweiten Zapfenlöchern 112, 112' und mit einem Paar von Positionierschlitzen 13, 13' versehen, wobei die Positionierschlitze 13, 13' angepasst an die zweiten Zapfenlöcher 112, 112' ausgebildet sind. Der Hauptkörper 11 ist unten an seinen beiden Seiten mit ein Paar von einander gegenüberliegenden dritten Zapfenlöchern 114, 114' versehen. Die Rückstelleinheit 14 ist schwenkbar an einem in die dritten Zapfenlöcher 114, 114' eingeführten, zweiten Drehzapfen 17 angeordnet. Der Hauptkörper 11 ist an seiner Innenwand mit einem Anschlagvorsprung 113 versehen.

Die Oberschenkel-Verbindungsvorrichtung 2 besitzt einen Verbindungsabschnitt 21, der oben mit einem ersten Gewindeloch 211 versehen ist. Ein Oberschenkelarretierelement 212 ist in das erste Gewindeloch 211 einschraubbar, wobei das Oberschenkelarretierelement 212 mit dem Oberschenkel verbunden ist. Ausgehend von der Unterfläche des Verbindungsabschnitts 21 erstreckt sich ein Sockelabschnitt 22, der mit einem ersten Einstellgewindeloch 221 versehen ist, in das ein erster Einstellgewindebolzen 224 einschraubbar ist. Der erste Einstellgewindebolzen 224 ist innen aus elastischem Material hergestellt. Kommt die Unterfläche des ersten Einstellgewindebolzens 224 infolge einer bewegungsbedingten Verschwenkung des Oberschenkel-Verbindungselements 2 relativ zum Bremsbügel 4 mit der Umfangsfläche des Bremsbügels 4 in Berührung, ergibt sich dann die stoßdämpfende Wirkung. Ausgehend von beiden Seiten eines Endes des Sockelabschnitts 22 erstreckt sich jeweils ein zweiter Befestigungsabschnitt 222, 222'. An einem anderen Ende des Sockelabschnitts 22 ist ein Verbindungsabschnitt 223 ausgebildet. Eine erste Aufnahmenut 2221 ist von den zweiten Befestigungsabschnitten 222, 222' und dem Sockelabschnitt 22 begrenzt. Die Befestigungsabschnitte 222, 222' besitzen jeweils ein viertes Arretierloch 2222 bzw. 2222'. Der Verbindungsabschnitt 223 weist ein Verbindungsloch 2231 auf, das über einen ersten Drehzapfen 16 schwenkbar mit dem anderen Ende des Antriebselements 15 verbunden ist.

Das Bremselement 3 ist im Aufnahmeraum 111 vorgesehen. Außerdem weist das Bremselement 3 ein erstes Arretierloch 31, ein zweites Arretierloch 32, ein erstes Arretierelement 35 und ein zweites Arretierelement 36 auf. Das erste und das zweite Arretierelement 35, 36 greifen in das erste bzw. das zweite Arretierloch 31, 32 ein. Das erste Arretierelement 35 umfasst einen ersten Drehstift 351 und einen ersten Gewindestift 352, während das zweite Arretierelement 36 einen zweiten Drehstift 361 und einen zweiten Gewindestift 362 besitzt. Das Bremselement 3 ist mit einer Gleitschiene 33 versehen, die über ein zweites Einstellgewindeloch 332 verfügt. Außerdem weist die Gleitschiene 33 eine Anschlagfläche 331 auf. In das zweite Einstellgewindeloch 332 ist ein zweiter Einstellgewindebolzen 333 einschraubbar, der mit einem Anlageelement 334 in Berührung kommt.

Der Bremsbügel 4 befindet sich im Aufnahmeraum 111 und besitzt ein erstes Zapfenloch 41. Ausgehend von beiden Seiten des ersten Zapfenlochs 41 erstreckt sich ein erstes Befestigungsabschnitt 42 bzw. 42'. Von den beiden Befestigungsabschnitten 42, 42' und dem ersten Zapfenloch 41 ist eine zweite Aufnahmenut 422 begrenzt. Die Befestigungsabschnitte 42, 42' weisen jeweils ein drittes Arretierloch 421 bzw. 421' auf. An der äußeren Begrenzungsfläche des ersten Zapfenlochs 41 ist ein Anlageabschnitt 43 ausgebildet, der in der zweiten Aufnahmenut 422 vorgesehen ist. Der Anlageabschnitt 43 weist eine Anlagenut 431 auf, in der ein Anlageelement 334 aufnehmbar ist.

Der Bremszapfen 5 wird in das erste Zapfenloch 41 des Bremsbügels 4 eingeführt. An beiden Enden des Bremszapfens 5 ist jeweils ein Positionierabschnitt 52 bzw. 52' angeordnet. Die beiden Positionierabschnitte 52, 52' sind in der Baugröße und der Bauform angepasst an die Positionierschlitze 13, 13' der Unterschenkel-Verbindungsvorrichtung 1 ausgebildet. Die beiden Positionierabschnitte 52, 52' sind mit einem zweiten Gewindeloch 51 versehen, das durch beide Enden des Bremszapfens 5 hindurch verläuft.

Beim Zusammenbau wird der Bremszapfen 5 in das erste Zapfenloch 41 des Bremsbügels 4 eingeführt. Danach wird ein Paar von Gewindestangen 53, 53' (Schrauben) durch die zweiten Zapfenlöcher 112 bzw. 112' hindurch in das zweite Gewindeloch 51 des Bremszapfens 5 eingeschraubt Hierdurch ergibt sich die Befestigung des Bremszapfens 5 an der Unterschenkel-Verbindungsvorrichtung 1. Außerdem kann der Bremsbügel 4 gegenüber dem Bremszapfen 5 geschwenkt werden. Dann wird ein Ende des Bremselements 3 in die zweite Aufnahmenut 422 des Bremsbügels 4 eingesetzt, derart, dass das Bremselement 3 gegen den Anlageabschnitt 43 des Bremsbügels 4 anliegt. Der zweite Drehstift 361 wird durch das dritte Arretierloch 421, 421' des Bremsbügels 4 und durch das zweite Arretierloch 32 des Bremselements 3 hindurchgeführt, während der zweite Gewindestift 362 ein viertes Gewindeloch 342 des Bremselements 3 durchsetzt und in eine zweite Nut 363 des zweiten Drehstifts 361 eingreift. Das andere Ende des Bremselements 3 wird in die ersten Aufnahmenut 2221 der Oberschenkel-Verbindungsvorrichtung 2 eingesetzt, wobei der erste Drehstift 351 durch das vierte Arretierloch 2222 bzw. 2222' der Oberschenkel-Verbindungsvorrichtung 2 sowie das erste Arretierloch 31 des Bremselements 3 hindurchgeführt wird. Außerdem durchsetzt der erste Gewindestift 352 ein drittes Gewindeloch 341 des Bremselements 3 und greift in eine erste Nut 353 des ersten Drehstifts 351 ein.

Ein Ende des Antriebselements 15 ist schwenkbar mit dem Verbindungsabschnitt 223 der Oberschenkel-Verbindungsvorrichtung 2 verbunden, während das andere Ende desselben mit der Rückstelleinheit 14 verbunden ist. Die Rückstelleinheit 14 ist im Aufnahmeraum 111 der Unterschenkel-Verbindungsvorrichtung 1 untergebracht und schwenkbar an der Unterschenkel-Verbindungsvorrichtung 1 angeordnet. Beim Abwinkeln des Prothesengelenks bietet die Rückstelleinheit 14 eine Rückstellkraft, mit der der Prothesenunterschenkel in seine Ausgangslage zurückkehrt.

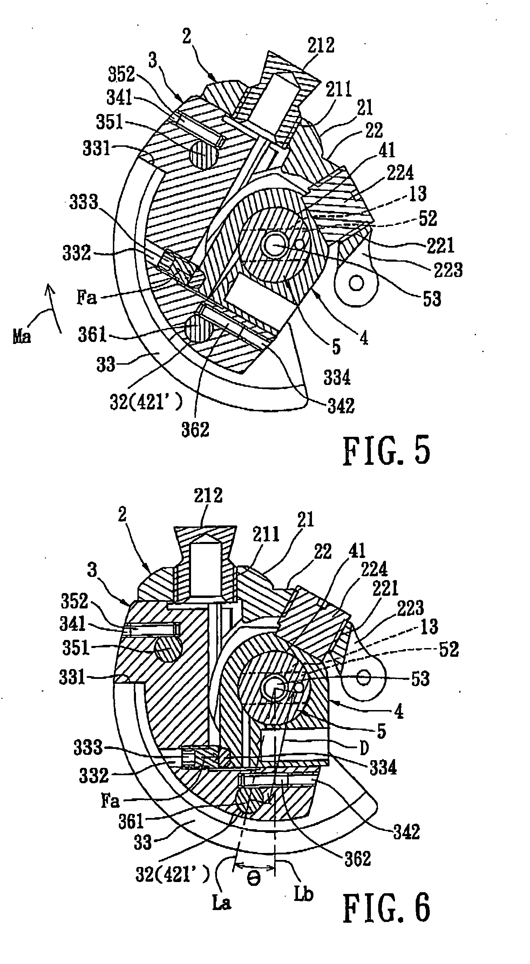

Nachfolgend wird auf Fig. 5 Bezug genommen. Befindet sich der Massenschwerpunkt des Körperbehinderten hinter dem Prothesengelenk, erzeugt das Körpergewicht gegenüber dem Bremszapfen 5 ein Drehmoment Ma im Uhrzeigersinn, wodurch der zweite Einstellgewindebolzen 333 des Bremselements 3 gegen das in der Anlagenut 431 des Bremsbügels 4 befindliche Anlageelement 334 anliegt und somit eine Kontaktkraft Fa erzeugt. Denn bei Anliegen des Drehmoments Ma kommt es zu einer Drehung des Bremselements 3 um den Drehstift 361 und damit zu einer Bewegung des Bremselements relativ zum Bremsbügel 4, so dass infolge der Lage des Drehstifts unterhalb des Einstellgewindebolzens 333 dieser gegen das Anlageelement 334 gedrückt wird, wodurch wiederum die Kontaktkraft Fa erzeugt wird. Mit der Kontaktkraft Fa lässt sich die Begrenzungsfläche des ersten Zapfenlochs 41 gegen die Außenwand des Bremszapfens 5 drücken. Damit wird vermieden, dass der Bremsbügel 4 um den Bremszapfen 5 schwenkt. Eine Seite des Anlageelements 334 ist flach ausgebildet und dient der Berührung mit dem zweiten Einstellgewindebolzen 333, während die andere Seite desselben als Halbkreisbogen ausgebildet ist, der mit der Anlagenut 431 in Berührung kommt. Auf diese Weise kann der Kontaktkraft Fa gleichmäßig auf den Bremsbügel 4 einwirken, derart, dass die Begrenzungsfläche des ersten Zapfenlochs 41 gegen die Außenwand des Bremszapfens 5 gedrückt wird. Außerdem ist die Kontaktstraffheit des zweiten Einstellgewindebolzens 333 mit dem Anlageelement 334 einstellbar, sodass die Begrenzungsfläche des ersten Zapfenlochs 41 noch stärker gegen die Außenwand des Bremszapfens 5 gedrückt werden kann. Darüber hinaus tritt der Verschleiß am Anlageelement 334 nur schwierig auf, wodurch eine erhöhte Standzeit des Prothesengelenks erzielbar ist.

Vor oder gleichzeitig mit dem Verschwenken des Bremselements 3 um den Drehstift 361 relativ zum Bremsbügel 4 kommt es zu einer weiteren Relativbewegung. Denn infolge des anliegenden Drehmoments Ma verschwenkt auch die Oberschenkel-Verbindungsvorrichtung 2 um den Drehzapfen 351, so dass es zu einer Bewegung des Oberschenkel-Verbindungselements 2 relativ zum Bremselement 3, aber auch relativ zum Bremsbügel 4 kommt. Hierbei wird der Einstellgewindebolzen 224 gegen den Bremsbügel gedrückt. Infolge der elastischen, dämpfenden Eigenschaft des Einstellgewindebolzens kommt es zu einer gedämpften Bewegung. Das heißt, dass bei einer Belastung des Gelenks, bei der es zum Aufbau eines im Uhrzeigersinn gerichteten Drehmoments kommt, zwei Drehbewegungen einsetzen, nämlich eine erste Drehung des Oberschenkel-Verbindungselements 2 um den Drehstift 351 relativ zum Bremselement 3 (wobei diese Bewegung gedämpft erfolgt, wenn ein dämpfender Einstellgewindebolzen vorgesehen ist, was nicht zwingend der Fall sein muss), sowie eine zweite Drehung des Bremselements 3 um den Drehstift 361 relativ zum Bremsbügel 4, wobei es zum Verklemmen des Bremszapfens 5 im Bremsbügel 4; also einer Sperrung des Gelenks kommt.

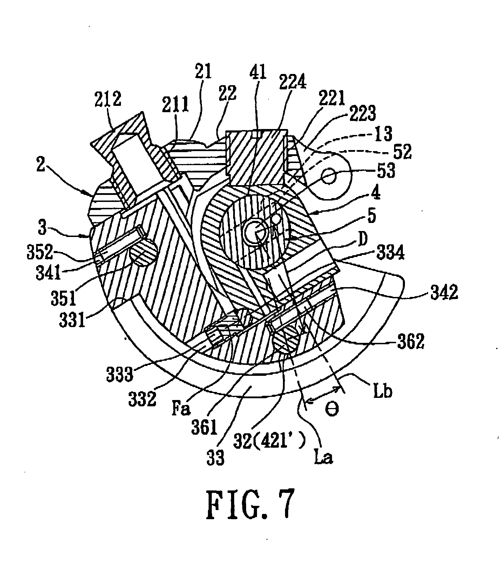

Es wird dann auf Fig. 6 und Fig. 7 Bezug genommen. Befindet sich der Massenschwerpunkt des Körperbehinderten vor dem Prothesengelenk, erzeugt das Körpergewicht gegenüber dem Bremszapfen 5 kein Drehmoment, wodurch der Bremsbügel 4 um den Bremszapfen 5 schwenkbar ist. Außerdem tritt keine Störung auf, was dem Körperbehinderten eine erhöhte Gehflexibilität verleiht. Der Winkel θ zwischen der von dem Mittelpunkt des dritten Arretierlochs 421, 421' und dem Mittelpunkt des Zapfenlochs 41 gebildeten Verbindungslinie La und der Senkrechtlinie Lb des Mittelpunkts des ersten Zapfenlochs 41 sowie der Abstand D zwischen dem Mittelpunkt des dritten Arretierlochs 421, 421' und dem Mittelpunkt des ersten Zapfenlochs 41 stehen damit im Zusammenhang, wie die Begrenzungsfläche des ersten Zapfenlochs 41 gegen die Außenwand des Bremszapfens 5 gedrückt wird. Im dargestellten Ausführungsbeispiel liegt der Winkel θ im Bereich von 15° bis 25°. Je größer der Abstand D ist, desto besser wird die Druckkung der Begrenzungsfläche des ersten Zapfenlochs 41 gegen die Außenwand des Bremszapfens 5.

Macht der Körperbehinderte einen Schritt, befindet sich der Massenschwerpunkt des Körperbehinderten hinter dem Prothesengelenk [siehe Fig. 5]. Nun wird die Begrenzungsfläche des ersten Zapfenlochs 41 gegen die Außenwand des Bremszapfens 5 gedrückt. Damit wird vermieden, dass der Bremsbügel 4 um den Bremszapfen 5 schwenkt, bis der Fuß mit dem Boden in Berührung kommt und der Körper nach vorne verschoben wird. Im Zeitraum zwischen dem Zeitpunkt, dass der Massenschwerpunkt des Körperbehinderten vor das Prothesengelenk verschoben wird [siehe Fig. 6] und dem Zeitpunkt, dass die Spitze des Fußes vom Boden entfernt wird [siehe Fig. 7], kann der Bremsbügel 4 um den Bremszapfen 5 geschwenkt werden. Auf diese Weise geschieht keine abnormale Abbiegung des Prothesengelenks im Zeitraum zwischen dem Zeitpunkt, dass der Körperbehinderte einen Schritt macht, und dem Zeitpunkt auf, dass der Fuß mit dem Boden in Berührung kommt und der Massenschwerpunkt des Körperbehinderten vor das Prothesengelenk verschoben wird. Damit ist eine sichere Verwendung gewährleistet. Darüber hinaus kann das Prothesengelenk im Zeitraum zwischen dem Zeitpunkt, dass der Körper des Körperbehinderten nach vorne verschoben wird, und dem Zeitpunkt, dass er den nächsten Schritt macht, frei abgewinkelt und geschwenkt werden. Hierdurch ergibt sich eine leichtgängige Gehbewegung.

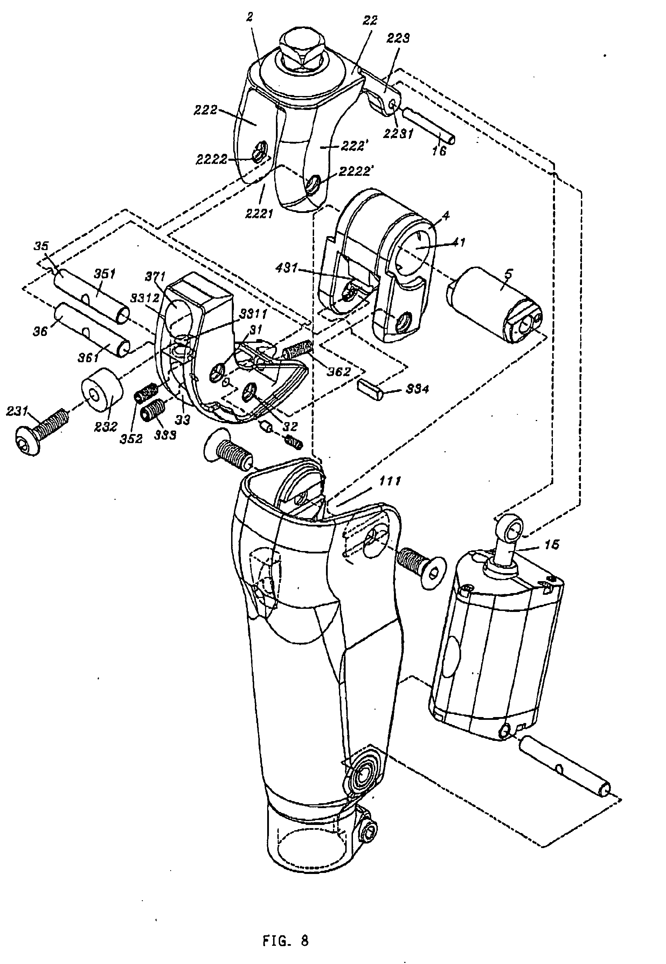

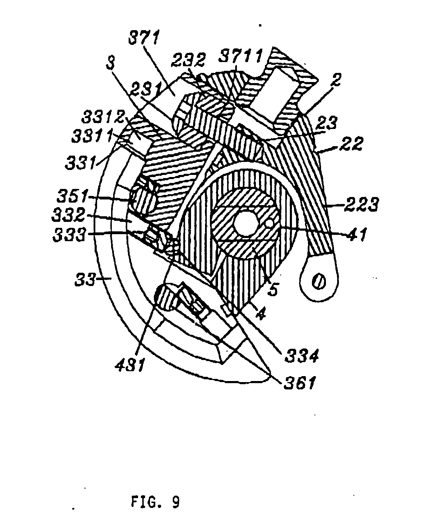

In den Fig.8 und 9 ist eine zweite Ausführungsform eines erfindungsgemäßen Prothesengelenks beschrieben. Die Oberschenkel-Verbindungsvorrichtung 2 besitzt einen Sockelabschnitt 22. Unterhalb des Sockelabschnitt befindet sich ein Gewindeloch 23, in das eine Einstellspannschraube 231 einschraubbar ist. Die Einstellspannschraube 231 befestigt einen elastischen Puffer 232, welcher mit der Unterseite im Kontakt zum Bremselement 3 steht. Der Puffer 232 ist austauschbar, so dass ohne weiteres ein in seiner Härte bzw. seinem Dämpfungsverhalten der gegebenen realen Anforderung beim Tragen der Prothese abgestimmter Puffer eingesetzt werden kann. Bei Kontakt des elastischen Puffers 232 mit dem Bremselement 3 bzw. einem Verschwenken der Oberschenkel-Verbindungsvorrichtung 2 um den Drehstift 351 kommt infolge der Verkippung der Einstellspannschraube 231, die mit der Oberschenkel-Verbindungsvorrichtung 2, weil in dieser fest eingeschraubt, bewegt wird, zu einer Deformation des Puffers 232 und hierüber zu einer dämpfenden Wirkung.

Ausgehend von beiden Seiten eines Endes des Sockelabschnitts 22 erstreckt sich jeweils ein zweiter Befestigungsabschnitt 222, 222'. An einem anderen Ende des Sockelabschnitts 22 ist ein Verbindungsabschnitt 223 ausgebildet. Eine erste Aufnahmenut 2221 ist von den zweiten Befestigungsabschnitten 222, 222' und dem Sockelabschnitt 22 begrenzt. Die Befestigungsabschnitte 222, 222' besitzen jeweils Arretierlöcher 2222 bzw. 2222'. Der Verbindungsabschnitt 223 weist ein Verbindungsloch 2231 auf, das über einen ersten Drehzapfen 16 schwenkbar mit dem anderen Ende des Antriebselements 15 verbunden ist.

Das Bremselement 3 ist im Aufnahmeraum 111 vorgesehen. Außerdem weist das Bremselement 3 ein erstes Arretierloch 31, ein zweites Arretierloch 32, ein erstes Arretierelement 35 und ein zweites Arretierelement 36 auf. Das erste und das zweite Arretierelement 35, 36 greifen in das erste bzw. das zweite Arretierloch 31, 32 ein. Das erste Arretierelement 35 umfasst einen ersten Drehstift 351 und einen ersten Gewindestift 352, während das zweite Arretierelement 36 einen zweiten Drehstift 361 und einen zweiten Gewindestift 362 besitzt. Das Bremselement 3 ist mit einer Gleitschiene 33 versehen, die über ein zweites Einstellgewindeloch 332 verfügt. Außerdem weist die Gleitschiene 33 eine Anschlagfläche 331 auf. In der Anschlagfläche 331 ist eine Bohrung 3311, in dieser Bohrung 3311 wird ein elastischer Körper 3312 fixiert. Oberhalb der Anschlagfläche 331 der Gleitschiene 33 ist eine Eintiefung, gegebenenfalls mit einer bodenseitigen eine Ausbuchtung 371, für die Einstellspannschraube 231 und den elastischen Puffer 232 ausgeführt. Dabei berührt nur der Boden oder die Anlagefläche 3711 der gegebenenfalls vorgesehenen Ausbuchtung 371 den elastischen Puffer 232. In das zweite Einstellgewindeloch 332 ist ein zweiter Einstellgewindebolzen 333 einschraubbar, der mit einem Anlageelement 334 in Berührung kommt.

Befindet sich der Massenschwerpunkt des Körperbehinderten hinter dem Prothesengelenk, erengt das Körpegewicht gegenüber dem Bremszapfen 5 ein Drehmoment Ma im Uhrzeigersinn. Dadurch schwenkt die Oberschenkel-Verbindungsvorrichtung 2 um den ersten Drehstrift 351. Die Bewegung wird durch die Anlage des elastischen Puffers 232 am Bremselement 3 gedämpft und begrenzt, da die Einstellschraube 231 den Puffer 232 durchsetzt und diesen beim Verschwenken verformt, so dass der Puffer eine die Schwenkbewegung dämpfende Rückstellkraft aufbaut. Anschließend schwenkt sich das Bremselement 3 um den zweiten Drehstrift 361, wodurch der zweite Einstellgewindebolzen 333 des Bremselements 3 gegen das in der Anlagenut 431 des Bremsbügel 4 befindliche Anlageelement 334 anliegt und somit eine Kontaktkraft Fa erzeugt. Denn bei Anliegen des Drehmoments Ma kommt es zu einer Drehung des Bremselements 3 um den Drehstift 361 und damit zu einer Bewegung des Bremselements relativ zum Bremsbügel 4, so dass infolge der Lage des Drehstifts unterhalb des Einstellgewindebolzens 333 dieser gegen das Anlageelement 334 gedrückt wird, wodurch wiederum die Kontaktkraft Fa erzeugt wird. Mit der Kontaktkraft Fa lässt sich die Begrenzungsfläche des ersten Zapfenlochs 41 gegen die Außenwand des Bremszapfens 5 drücken. Damit wird vermieden, dass der Bremsbügel 4 um den Bremszapfen 5 schwenkt.

Wird die Unterschenkel-Verbindungsvorrichtung ausgehend von einer abgewinkelten Stellung in die Ausgangsstellung bewegt, so wird hierbei der Anschlagvorsprung 113 in der kreisbogenförmigen Gleitschiene 33 geführt, bis die Bewegung durch Anschlagen des Anschlagvorsprungs 113 an der Anschlagfläche 331 begrenzt wird. Dieses Anschlagen ist über den elastischen Körper 3312, gegen den der Anschlagvorsprung 113 läuft, gedämpft.

Die Erfindung bzw. beide Ausführungsformen zeichnen sich grundlegend dadurch aus, dass infolge der Schwenklagerung der Oberschenkel-Verbindungsvorrichtung 2 am Bremselement und der Schwenklagerung des Bremsbügels 4 am Bremselement zwei separate Drehbewegungen möglich sind, nämlich ein Verschwenken der Oberschenkel-Verbindungsvorrichtung 2 relativ zum Bremselement 3, welche Schwenkbewegung durch ein etwaiges vorhandenes Dämpfungselement (224 oder 232) gedämpft sein kann, und ein Verschwenken des Bremselements 3 relativ zum Bremsbügel 4, welche Bewegung zum Verklemmen des Bremszapfens 5 im Bremsbügel 4 und damit zu einer Arretierung des Gelenks insgesamt führt.

Obwohl die Erfindung in Bezug auf zwei Beispiele beschrieben wurde, welche derzeit als praktikabelste und bevorzugte Ausführungsformen betrachtet werden, versteht es sich, dass die Erfindung nicht auf die offenbarten Ausführungsbeispiele beschränkt ist. Im Gegenteil sollen verschiedene Modifikationen und ähnliche Anordnungen abgedeckt werden, deren Merkmale im Schutzbereich der beigefügten Ansprüche liegen.

### Bezugszeichenliste

- 1a: Unterschenkel-Verbindungsvorrichtung
- 11a: Befestigungszapfen
- 2a: Puffervorrichtung
- 3a: Bremsvorrichtung
- 31a: Feder
- 4a: Oberdeckel
- 41a: Vorderarm
- 42a: Hinterarm
- 43a: Einstellvorrichtung
- 44a: Zapfen
- 45a: Keilstück
- F2: nach unten gerichtete Kraft
- M: Betätigungsmoment
- FS: Aufspannkraft
- 1: Unterschenkel-Verbindungsvorrichtung
- 11: Hauptkörper
- 111: Aufnahmeraum
- 112: zweites Zapfenlochs
- 112': zweites Zapfenloch
- 113: Anschlagvorsprung
- 114: drittes Zapfenloch
- 114': drittes Zapfenloch
- 12: Sockel
- 121: Unterschenkel-Befestigungsöffnung
- 122: Uriterschenkel-Arretiemut
- 13: Positionierschlitz
- 13': Positionierschlitz
- 14: Rückstelleinheft
- 15: Antriebselement
- 16: erster Drehzapfen
- 17: zweiter Drehzapfen
- 2: Oberschenkel-Verbindungsvorrichtung
- 21: Verbindungsabschnitt
- 211: erstes Gewindeloch
- 212: Oberschenkelarretierelement
- 22: Sockelabschnitt
- 221: erstes Einstellgewindeloch
- 222: zweiter Befestigungsabschnitt
- 222': zweiter Befestigungsabschnitt
- 2221: erste Aufnahmenut
- 2222: viertes Arretierloch
- 2222': viertes Arretierloch
- 223: Verbindungsabschnitt
- 2231: Verbindungsloch
- 224: erster Einstellgewindebolzen
- 23: Gewindeloch
- 231: Einstellspannschraube
- 232: elastischer Puffer
- 3: Bremselement
- 31: erstes Arretierloch
- 32: zweites Arretierloch
- 33: Gleitschiene
- 331: Anschlagfläche
- 3311: Bohrung Anschlagfläche
- 3312: elastischer Körper
- 332: zweites Einstellgewindeloch
- 333: zweiter Einstellgewindebolzen
- 334: Anlageelement
- 341: drittes Gewindeloch
- 342: viertes Gewindeloch
- 35: erstes Arretierelement
- 351: erster Drehstift
- 352: erster Gewindestift
- 353: erste Nut
- 36: zweiter Drehstift
- 361: zweiter Drehstift
- 362: zweiter Gewindestift
- 363: zweite Nut
- 371: Ausbuchtung
- 3711: Anlagefläche
- 4: Bremsbügel
- 41: erstes Zapfenloch
- 42: Befestigungsabschnitt
- 42': Befestigungsabschnitt
- 421: drittes Arretierloch
- 421': drittes Arretierloch
- 422: zweite Aufnahmenut
- 43: Anlageabschnitt
- 431: Anlagenut
- 5: Bremszapfen
- 51: zweites Gewindeloch
- 52: Positionierabschnitt
- 52': Positionierabschnitt
- 53: Gewindestange
- 53': Gewindestange
- Ma: Drehmoment
- Fa: Kontaktkraft
- La: Verbindungslinie
- Lb: Senkrechtlinie Winkel
- D: Abstand

## Patentansprüche

1. Störungsfreies Sicherheits-Prothesengelenk, aufweisend:
eine Unterschenkel-Verbindungsvorrichtung (1), die einen Hauptkörper (11), einen Sockel (12), ein Antriebselement (15) und eine Rückstelleinheit (14) umfasst, wobei ein Aufnahmeraum (111) von der Innenwand des Hauptkörpers (11) begrenzt wird, und wobei sich der Sockel (12) ausgehend von einem Ende des Hauptkörpers (11) erstreckt, und wobei die Rückstelleinheit (14) im Aufnahmeraum (111) vorgesehen ist, und wobei ein Ende der Rückstelleinheit (14) mit dem Antriebselement (15) verbunden ist, während das andere Ende derselben schwenkbar mit dem Hauptkörper (11) verbunden ist;
eine Oberschenkel-Verbindungsvorrichtung (2), die einen Verbindungsabschnitt (21) und einen Sockelabschnitt (22) besitzt, wobei der Verbindungsabschnitt (21) an seinem einen Ende mit einem Oberschenkelarretierelement (212) versehen ist, während der Sockelabschnitt (22) an einem anderen Ende des Verbindungsabschnitts (21) angeordnet ist, und wobei die Rückstelleinheit (14) an einem Ende des Antriebselements (15) befestigt ist, während der Sockelabschnitt (22) schwenkbar an dem anderen Ende desselben angeordnet ist;
ein Bremsbügel (4), der sich im Aufnahmeraum (111) befindet, wobei der Bremsbügel (4) ein erstes Zapfenloch (49) und einen ersten Befestigungsabschnitt (42) aufweist, und wobei der erste Befestigungsabschnitt (42) mit einem dritten Arretierloch (421) versehen ist; und
einen Bremszapfen (5), der durch das Zapfenloch (41) hindurch verläuft und an der Unterschenkel-Verbindungsvorrichtung (1) befestigt ist;
**dadurch gekennzeichnet, daß** ein Bremselement (3), das im Aufnahmeraum (111) vorgesehen und an der Oberschenkel-Verbindungsvorrichtung (2) befestigt ist mit dem dritten Arretierloch (421) des Bremsbügels verbundet ist.

2. Störungsfreies Sicherheits-Prothesengelenk nach Anspruch 1,
**dadurch gekennzeichnet,**
**dass** der Sockelabschnitt (22) mit einem ersten Einstellgewindeloch (221) versehen ist, in das ein erster Einstellgewindebolzen (224) einschraubbar ist, und wobei der erste Einstellgewindebolzen (224) mit dem Bremsbügel (4) in Berührung kommt.

3. Störungsfreies Sicherheits-Prothesengelenk nach Anspruch 2, **dadurch gekennzeichnet,**
**dass** der Einstellgewindebolzen (224) teilweise aus einem elastischen stoßdämpfenden Material besteht.

4. Störungsfreies Sicherheits-Prothesengelenk nach Anspruch 1,
**dadurch gekennzeichnet,**
**dass** unterhalb des Sockelabschnitts (22) ein Gewindeloch (23) vorgesehen ist, in das eine durch das Bremselement (3) verlaufende Einstellspannschraube (231) eingeschraubt ist, über welche Einstellspannschraube (231) ein elastischer Puffer (232) an dem Bremselement (3) fixiert ist.

5. Störungsfreies Sicherheits-Prothesengelenk nach Anspruch 4,
**dadurch gekennzeichnet,**
**dass** am Bremselement (3) eine Eintiefung vorgesehen ist, deren Boden vorzugsweise eine Ausbuchtung (371) aufweist, in welche Eintiefung der Puffer (232) eingesetzt ist, der über die in das Gewindeloch (23) des Sockelabschnitts (22) eingeschraubte Einstellspannschraube (231) gegen den Boden, insbesondere in die Ausbuchtung (371) gedrückt wird.

6. Störungsfreies Sicherheits-Prothesengelenk nach Anspruch 3, 4 oder 5,
**dadurch gekennzeichnet,**
**dass** die Oberschenkel-Verbindungsvorrichtung (2) drehbar mit dem Bremselement (3) und das Bremselement (3) drehbar mit dem Bremsbügel (4) verbunden ist, wobei die Drehbewegung der Oberschenkel-Verbindungsvorrichtung (2) relativ zum Bremselement (3) über den Einstellgewindebolzen (224) oder den Puffer (232) gedämpft ist und die Drehbewegung der Bremsbügels (4) relativ zum Bremselement (3) zum Verklemmen des Bremszapfens (5) in dem Zapfenloch (41) führt.

7. Störungsfreies Sicherheits-Prothesengelenk nach einem der vorangehenden Ansprüche,
**dadurch gekennzeichnet,**
**dass** der Bremsbügel (4) eine Anlagenut (431) aufweist, in der ein Anlageelement (334) aufgenommen ist, wobei das Bremselement (3) einen zweiten Einstellgewindebolzen (333) besitzt, der gegen das Anlageelement (334) anliegt.

8. Störungsfreies Sicherheits-Prothesengelenk nach Anspruch 7,
**dadurch gekennzeichnet,**
**dass** eine Seite des Anlageelements (334) flach ausgebildet ist, während die andere Seite desselben als Halbkreisbogen ausgebildet ist.

9. Störungsfreies Sicherheits-Prothesengelenk nach einem der vorangehenden Ansprüche,
**dadurch gekennzeichnet,**
**dass** sich ein zweiter Befestigungsabschnitt (222) ausgehend von dem Sockelabschnitt (22) erstreckt, wobei ein Ende des zweiten Befestigungsabschnitts (222) mit einer ersten Aufnahmenut (2221) versehen ist, während der erste Befestigungsabschnitt (42) eine zweite Aufnahmenut (422) besitzt, und wobei ein Ende des Bremselements (3) in der ersten Aufnahmenut (2221) vorgesehen ist, während sich das andere Ende des Bremselements (3) in der zweiten Aufnahmenut (422) befindet.

10. Störungsfreies Sicherheits-Prothesengelenk nach Anspruch 9, **dadurch gekennzeichnet,**
**dass** ein Drehstift (351), über den die Oberschenkel-Verbindungsvorrichtung (2) mit dem Bremselement (3) schwenkbar verbunden ist, ein im zweiten Befestigungsabschnitt (222) vorgesehenes viertes Arretierloch (2222,2222') sowie ein am in der Aufnahmenut (2221) aufgenommenen Ende des Bremselements (3) vorgesehenes erstes Arretierloch (31) durchsetzt, und dass ein Drehstift (361), über den der Befestigungsbügel (4) mit dem Bremselement (3) schwenkbar verbunden ist, ein im ersten Befestigungsabschnitt (42) drittes Arretierloch (421,421') sowie ein am in der Aufnahmenut (422) aufgenommenen Ende des Bremselements (3) vorgesehenes zweites Arretierloch (32) durchsetzt.

11. Störungsfreies Sicherheits-Prothesengelenk nach einem der vorangehenden Ansprüche,
**dadurch gekennzeichnet,**
**dass** das Bremselement (3) mit einer Gleitschiene (33) versehen ist, wobei der Hauptkörper (11) an seiner Innenseite mit einem Anschlagvorsprung (113) versehen ist, der in der Gleitschiene (33) aufnehmbar ist, und wobei eine Anschlagfläche (331) an einem Ende der Gleitschiene (33) ausgebildet ist, und wobei der Anschlagvorsprung (113) gegen die Anschlagfläche (331) anschlägt.

12. Störungsfreies Sicherheits-Prothesengelenk nach Anspruch 10, **dadurch gekennzeichnet,**
**dass** in der Anschlagfläche (331) eine Bohrung (3311) vorgesehen ist, in der ein elastischer Körper (3312), der das Anschlagen des Anschlagvorsprungs (113) dämpft, aufgenommen ist.

13. Störungsfreies Sicherheits-Prothesengelenk nach einem der vorangehenden Ansprüche,
**dadurch gekennzeichnet,**
**dass** die von dem Mittelpunkt des am Bremsbügel (4) vorgesehenen dritten Arretierlochs (421) und dem Mittelpunkt des am Bremsbügel (4) vorgesehenen Zapfenlochs (41) gebildete verbindungslinie in einem Winkel von 15° bis 25° zur Senkrechtlinie des Mittelpunkts des ersten Zapfenlochs (41) steht.

## Claims

1. A trouble-free safety prosthesis joint, comprising:
a lower-leg connecting device (1), which provides a main element (11), a base (12), a driving element (15) and a re-setting unit (14), wherein a retaining chamber (111) is limited by the internal wall of the main element (11), and wherein the base (12) extends outwards from one end of the main element (11), and wherein the re-setting unit (14) is provided in the retaining chamber (111), and
wherein one end of the re-setting unit (14) is connected to the driving element (15), while the other end of the same is connected in a pivoting manner to the main element (11);
a femur connecting device (2), which provides a connecting portion (21) and a base portion (22), wherein the connecting portion (21) is provided at its one end with a femur locking element (212), while the base portion (22) is arranged at the other end of the connecting portion (21), and
wherein the re-setting unit (14) is attached at one end of the driving element, while the base portion (22) is arranged in a pivoting manner at the other end of the same;
a braking strap (4), which is disposed within the retaining chamber (111), wherein the braking strap (4) provides a first pivot borehole (41) and a first attachment portion (42), and wherein the first attachment portion (42) is provided with a third locking borehole (421); and
a braking pivot (5), which extends through the pivot borehole (41) and is attached to the lower-leg connecting device (1),
**characterised in that**
a braking element (3), which is provided in the retaining chamber (111) and is attached to the femur connecting device (2), is connected to the third locking borehole (421) of the braking strap.

2. The trouble-free safety prosthesis joint according to claim 1,
**characterised in that**
the base portion (22) is provided with a threaded adjustment borehole (221), into which a first threaded adjustment screw (224) can be screwed, and wherein the first threaded adjustment screw (224) comes into contact with the braking strap (4).

3. The trouble-free safety prosthesis joint according to claim 2,
**characterised in that**
the threaded adjustment screw (224) is made in part from an elastic, shock-absorbing material.

4. The trouble-free safety prostheses joint according to claim 1,
**characterised in that**
below the base portion (22), a threaded borehole (23) is provided, into which an adjustable tension screw (231) extending through the braking element (3) is screwed, by means of which adjustable tension screw (231), an elastic buffer (232) is fixed to the braking element (3).

5. The trouble-free safety prosthesis joint according to claim 4,
**characterised in that**,
on the braking element (3), an indentation is provided, the base of which preferably provides a recess (371), into which indentation the buffer (232), which is pressed via the adjustable tension screw (231) screwed into the threaded borehole (23) of the base portion (22) against the base, especially into the recess (371), is inserted.

6. The trouble-free safety prosthesis joint according to claim 3, 4 or 5,
**characterised in that**
the femur connecting device (2) is connected in a rotatable manner to the braking strap (4), wherein the rotary movement of the femur connecting device (2) relative to the braking element (3) is dampened via the threaded adjustment screw (224) or the buffer (232), and the rotary movement of the braking strap (4) relative to the braking element (3) leads to the clamping of the braking pivot (5) in the pivot borehole (41).

7. The trouble-free safety prosthesis joint according to any one of the preceding claims,
**characterised in that**
the braking strap (4) provides an engagement groove (431), in which an engagement element (334) is retained, wherein the braking element (3) provides a second threaded adjustment screw (333), which is in contact against the engagement element (334).

8. The trouble-free safety prosthesis joint according to claim 7,
**characterised in that**
one side of the engagement element (334) is formed flat, while the other side of the same is formed as a semicircular arch.

9. The trouble-free safety prosthesis joint according to any one of the preceding claims,
**characterised in that**
a second attachment portion (222) extends outwards from the base portion (22), wherein one end of the second attachment portion (222) is provided with a retaining groove (2221), while the first attachment portion (42) provides a second retaining groove (422), and wherein one end of the braking element (3) is provided in the first retaining groove (2221), while the other end of the braking element (3) is disposed in the second retaining groove (422).

10. The trouble-free safety prosthesis joint according to claim 9,
**characterised in that**
a pivot pin (351), by means of which the femur connecting device (2) is connected in a pivoting manner to the braking element (3), penetrates a fourth locking borehole (2222, 2222') provided in the second attachment portion (222) and a first locking borehole (31) provided at the end of the braking element (3) accommodated in the retaining groove (2221), and that a pivot pin (361), by means of which the attachment strap (4) is connected in a pivoting manner to the braking element (3), penetrates, a third locking borehole (421, 421') in the first attachment portion (42) and a second locking borehole (32) provided in the end of the braking element (3) accommodated in the retaining groove (422).

11. The trouble-free safety prosthesis joint according to claim 9,
**characterised in that**
the braking element (3) is provided with a sliding rail (33), wherein the main element (11) is provided on its internal side with a stopping projection (113), which can be retained in the sliding rail (33), and wherein a stopping surface (331) is formed at one end of the sliding rail (33), and wherein the stopping projection (113) stops against the stopping surface (331).

12. The trouble-free safety prosthesis joint according to claim 10,
**characterised in that**
in the stopping surface (331), a borehole (3311) is provided, in which an elastic element (3312) is retained which dampens the stopping of the stopping projection (113).

13. The trouble-free safety prosthesis joint according to any one of the preceding claims,
**characterised in that**
the connecting line formed from the central point of the third locking borehole (421) provided on the braking strap (4) and the central point of the pivot borehole (41) provided on the braking strap (4) is disposed at an angle of 15° to 25° relative to the perpendicular line of the central point of the first pivot borehole (41).

## Revendications

1. Prothèse d'articulation de sécurité sans contrainte, présentant :
un dispositif de liaison pour la jambe (1), qui comprend un corps principal (11), un socle (12), un élément d'entraînement (15) et une unité de rappel (14), un espace de réception (111) étant délimité par la paroi interne du corps principal (11), et le socle (12) s'étendant à partir d'une extrémité du corps principal (11), et l'unité de rappel (14) étant prévue dans l'espace de réception (111), et une extrémité de l'unité de rappel (14) étant reliée à l'élément d'entraînement (15), alors que l'autre extrémité de l'unité de rappel est reliée de façon basculante au corps principal (11) ;
un dispositif de liaison pour la cuisse (2), qui présente une section de liaison (21) et une section de socle (22), la section de liaison (21) étant dotée sur l'une de ses extrémités d'un élément d'arrêt de cuisse (212), alors que la section de socle (22) est disposée sur une autre extrémité de la section de liaison (21), et l'unité de rappel (14) étant fixée sur une extrémité de l'élément d'entraînement (15), alors que la section de socle (22) est disposée de façon basculante sur l'autre extrémité de cette section ;
un étrier de frein (4), qui se trouve dans l'espace de réception (111), l'étrier de frein (4) présentant un premier trou de pivot (41) et une première section de fixation (42), et la première section de fixation (42) étant dotée d'un troisième trou d'arrêt (421) ; et
un pivot de frein (5), qui s'étend à travers le trou de pivot (41) et est fixé sur le dispositif de liaison pour la jambe (1),
**caractérisée en ce qu'**un élément de frein (3), qui est prévu dans l'espace de réception (111) et est fixé sur le dispositif de liaison pour la cuisse (2), est relié au troisième trou d'arrêt (421) de l'étrier de frein.

2. Prothèse d'articulation de sécurité sans contrainte selon la revendication 1,
**caractérisée en ce que**
la section de socle (22) est dotée d'un premier trou fileté de réglage (221), dans lequel un premier boulon fileté de réglage (224) peut être vissé, et le premier boulon fileté de réglage (224) venant en contact avec l'étrier de frein (4).

3. Prothèse d'articulation de sécurité sans contrainte selon la revendication 2,
**caractérisée en ce que**
le boulon fileté de réglage (224) est en partie à base d'un matériau élastique amortisseur de choc.

4. Prothèse d'articulation de sécurité sans contrainte selon la revendication 1,
**caractérisée en ce**
**qu'**un trou fileté (23) est prévu au-dessous de la section de socle (22), trou dans lequel une vis de serrage de réglage (231) passant à travers l'élément de frein (3) est vissée, vis de serrage de réglage (231) par laquelle un tampon (232) élastique est fixé sur l'élément de frein (3).

5. Prothèse d'articulation de sécurité sans contrainte selon la revendication 4,
**caractérisée en ce**
**qu'**il est prévu sur l'élément de frein (3) une cavité dont le fond présente de préférence une échancrure (371), cavité dans laquelle le tampon (232) est inséré, lequel est pressé au moyen de la vis de serrage de réglage (231) vissée dans le trou fileté (23) de la section de socle (22) contre le fond, en particulier dans l'échancrure (371).

6. Prothèse d'articulation de sécurité sans contrainte selon la revendication 3, 4 ou 5,
**caractérisée en ce que**
le dispositif de liaison pour la cuisse (2) est relié de façon rotative à l'élément de frein (3) et l'élément de frein (3) est relié de façon rotative à l'étrier de frein (4), le mouvement de rotation du dispositif de liaison pour la cuisse (2) étant amorti par rapport à l'élément de frein (3) au moyen du boulon fileté de réglage (224) où du tampon (232) et le mouvement de rotation de l'étrier de frein (4) par rapport à l'élément de frein (3) entraînant le blocage du pivot de frein (5) dans le trou de pivot (41).

7. Prothèse d'articulation de sécurité sans contrainte selon l'une quelconque des revendications précédentes,
**caractérisée en ce que**
l'étrier de frein (4) présente une rainure d'appui (431), dans laquelle un élément d'appui (334) est réceptionné, l'élément de frein (3) possédant un deuxième boulon fileté de réglage (333), qui s'applique contre l'élément d'appui (334).

8. Prothèse d'articulation de sécurité sans contrainte selon la revendication 7,
**caractérisée en ce**
**qu'**un côté de l'élément d'appui (334) est conçu plat, alors que l'autre côté de l'élément est conçu en forme d'arc de demi-cercle.

9. Prothèse d'articulation de sécurité sans contrainte selon l'une quelconque des revendications précédentes,
**caractérisée en ce**
**qu'**une seconde partie de section de fixation (222) s'étend à partir de la section de socle (22), une extrémité de la seconde section de fixation (222) étant dotée d'une première rainure de logement (2221), alors que l'autre section de fixation (42) présente une seconde rainure de réception (422), et une extrémité de l'élément de frein (3) étant prévue dans la première rainure de la section (2221), alors que l'autre extrémité de l'élément de frein (3) se trouve dans la seconde rainure de réception (422).

10. Prothèse d'articulation de sécurité sans contrainte selon la revendication 9,
**caractérisée en ce**
**qu'**une tige rotative (351), par laquelle le dispositif de liaison pour la cuisse (2) est relié de façon pivotante à l'élément de frein (3), traverse un quatrième trou d'arrêt (2222, 2222') prévu dans la seconde section de fixation (222) et un premier trou d'arrêt (31) prévu sur l'extrémité, réceptionnée dans la rainure de réception (2221) de l'élément de frein (3), et en ce qu'une tige rotative (361), par laquelle l'étrier de fixation (4) est relié de façon pivotante à l'élément de frein (3), traverse un troisième trou d'arrêt (421, 421') prévu dans la première section de fixation (42) et un second trou d'arrêt (32) prévu sur l'extrémité, réceptionnée dans la rainure de réception (422), de l'élément de frein (3).

11. Prothèse d'articulation de sécurité sans contrainte selon l'une quelconque des revendications précédentes,
**caractérisée en ce que**
l'élément de frein (3) est doté d'une glissière (33), le corps principal (11) étant doté sur son côté intérieur d'une saillie de butée (113), laquelle peut être réceptionnée dans la glissière (33), et une surface de butée (331) étant réalisée sur une extrémité de la glissière (33), et la saillie de butée (113) butant contre la surface de butée (331).

12. Prothèse d'articulation de sécurité sans contrainte selon la revendication 10, **caractérisée en ce**
**qu'**il est prévu dans la surface de butée (331) un perçage (3311) dans lequel est réceptionné un corps (3312) élastique, qui amortit la butée de la saillie de butée (113).

13. Prothèse d'articulation de sécurité sans contrainte selon l'une quelconque des revendications précédentes,
**caractérisée en ce que**
la ligne de liaison, formée par le centre du troisième trou d'arrêt (421), prévu sur l'étrier de frein (4) et le centre du trou de pivot (41) prévu sur l'étrier de frein (4), forme un angle de 15 à 25° par rapport à la verticale du centre du premier trou de pivot (41).
